(19) **Europäisches Patentamt**

**European Patent Office**

**Office européen des brevets**

(11) **EP 1 362 029 B1**

(12) **FASCICULE DE BREVET EUROPEEN**

(45) Date de publication et mention
de la délivrance du brevet:
**14.09.2005 Bulletin 2005/37**

(21) Numéro de dépôt: **02703673.0**

(22) Date de dépôt: **07.02.2002**

(51) Int Cl.[7]: **C07C 409/38**, C07C 407/00,
C08K 5/14

(86) Numéro de dépôt international:
**PCT/FR2002/000475**

(87) Numéro de publication internationale:
**WO 2002/064555 (22.08.2002 Gazette 2002/34)**

(54) **PROCEDE DE PREPARATION D'UN HYDROXY-PEROXYESTER**

VERFAHREN ZUR HERSTELLUNG VON HYDROXY-PEROXYESTERN

HYDROXY-PEROXYESTER PREPARATION METHOD

(84) Etats contractants désignés:
**AT BE CH CY DE DK ES FI FR GB GR IE IT LI LU
MC NL PT SE TR**
Etats d'extension désignés:
**RO**

(30) Priorité: **13.02.2001 FR 0101921**

(43) Date de publication de la demande:
**19.11.2003 Bulletin 2003/47**

(73) Titulaire: **Arkema
92800 Puteaux (FR)**

(72) Inventeurs:
• **GRIMALDI, Sandra
F-69110 Sainte Foy-les-Lyon (FR)**
• **JEYARAJ, Gurusamy
Cuddalore 607001 (IN)**

(74) Mandataire: **Cabinet Hirsch
58, avenue Marceau
75008 Paris (FR)**

(56) Documents cités:
**EP-A- 0 126 216         GB-A- 1 106 953
US-A- 3 236 872         US-A- 3 446 831**

• **CHEMICAL ABSTRACTS, vol. 99, no. 20, 14
novembre 1983 (1983-11-14) Columbus, Ohio,
US; abstract no. 159030, HORIA, MIHAI ET AL:
"Tert-Butyl per-2-ethylhexanoate" XP002180202
& RO 79 161 B (INTREPRINDEREA CHIMICA
"DUDESTI", ROM.) 25 juin 1982 (1982-06-25)**
• **DATABASE WPI Section Ch, Week 198518
Derwent Publications Ltd., London, GB; Class
A60, AN 1985-107394 XP002180203 & JP 60
051169 A (NIPPON OILS & FATS CO LTD), 22
mars 1985 (1985-03-22)**

**Description**

La présente invention concerne un procédé de préparation d'un hydroxy-peroxyester pouvant être utilisé, notamment, dans des polymérisations, comme la polymérisation du chlorure de vinyle, ou dans le thermodurcissage des résines polyester.

De nombreux documents de l'art antérieur mentionnent la préparation de peroxyesters :

- la demande de brevet européen n°667339, qui a trait à des peroxydes insaturés et à des polymères obtenus à l'aide de ces peroxydes ;
- la demande de brevet européen n°126216, qui concerne des hydroxy-t-alkyl peroxyesters ayant des températures de demi-vie de 10 heures inférieures 75°C ;
- le brevet américain n° 3 624 124, qui se rapporte à des peresters d'alkyle tertiaire et d'hydroperoxyde tertiaire ;
- la demande de brevet européen n° 381 135, qui décrit des hydroxy-peroxydes ayant des températures de demi-vie de 10 heures comprises entre 85 et 100°C ;
- la demande de brevet européen n° 474 227, qui traite de peroxydes fonctionnalisés destinés à des réactions de polymérisation;
- le brevet américain n° 3 446 831, qui concerne un procédé de préparation de peroxyester du ter-amylhydroperoxide et
- le brevet américain n° 3 236 872, qui décrit un procédé de préparation d'hexylène glycol peroxide.

Cependant, dans tous ces documents, les peroxyesters sont préparés en présence d'un solvant et parfois d'un catalyseur de transfert de phase.

Lors de la préparation des peroxyesters possédant un groupement hydroxyle en position y par rapport à la fonction peroxyde O-O, des problèmes de décantation, c'est-à-dire de mauvaise séparation de la phase organique et de la phase aqueuse, peuvent être rencontrés.

Pour résoudre ces problèmes, on utilise alors un solvant qui permet d'accélérer le phénomène de décantation ; cependant, ce solvant pose un problème supplémentaire : il reste en partie au sein du peroxyester, ce qui est gênant lors de l'utilisation du peroxyester, à cause de la présence ultérieure de solvant dans le polymère.

Une autre solution consiste à attendre patiemment que la décantation s'effectue d'elle-même, ce qui peut prendre plusieurs jours.

Il a maintenant été découvert qu'il était possible d'obtenir une décantation rapide sans avoir recours à un solvant.

L'invention a donc pour objet un procédé de préparation d'un hydroxy-peroxyester comprenant la réaction d'un sel d'hydroxy-hydroperoxyde avec un halogénure d'acide ou un anhydride d'acide et dans lequel :

- le rapport molaire $R_b$ entre l'hydroxy-hydroperoxyde correspondant au sel d'hydroxy-hydroperoxyde et l'halogénure d'acide est compris entre 0,5 et 1,5 ; ou bien, le rapport molaire $R_b'$ entre l'hydroxy-hydroperoxyde correspondant au sel d'hydroxy-hydroperoxyde et l'anhydride d'acide est compris entre 1,0 et 3,0 ;
- le sel d'hydroxy-hydroperoxyde a été préalablement préparé par réaction de l'hydroxy-hydroperoxyde correspondant avec une base, selon un rapport molaire $R_a$ entre la base et l'hydroxy-hydroperoxyde compris entre 0,5 et 1,5 ; et
- ce procédé est mis en oeuvre en milieux aqueux en l'absence de solvant organique.

Un tel procédé offre donc les avantages suivants :

- la réaction du sel d'hydroperoxyde avec l'halogénure ou l'anhydride d'acide peut maintenant être mise en oeuvre en l'absence de solvant organique et, le cas échéant, de catalyseur de transfert de phase ;
- la décantation s'effectue rapidement, ce qui d'une part, permet de gagner du temps, et d'autre part, évite d'avoir à conserver certains peroxyesters peu stables dans des zones de stockage à basse température ;
- le peroxyester obtenu est exempt de tout solvant, on évite ainsi toute étape ultérieure d'évaporation ou de distillation du solvant, ce qui améliore le rendement ;
- la pureté du peroxyester obtenu est satisfaisante ;
- l'absence de solvant écarte en outre le risque d'introduire, dans le peroxyester ou le produit final, les impuretés éventuelles contenues dans le solvant ;
- le peroxyester peut être utilisé tel qu'obtenu, c'est-à-dire sans purification ultérieure, sans qu'il se produise, par exemple, lors de l'utilisation dans des réactions de polymérisation, des réactions parasites telles que des réactions de transfert, ou des problèmes de couleur ou d'odeur du polymère obtenu.

D'autres caractéristiques et avantages de l'invention apparaîtront à la lecture de l'exposé qui suit et qui est illustré par des exemples.

**Exposé détaillé de l'invention**

Le procédé de préparation selon l'invention peut donc être mis en oeuvre en phase aqueuse.

Il peut comprendre une étape préalable de préparation du sel d'hydroxy-hydroperoxyde par réaction d'un hydroxy-hydroperoxyde avec une base.

Ainsi, la synthèse du peroxyester peut être illustrée par le schéma réactionnel suivant :

Etape a) :

$$ROOH + base \rightarrow ROOM$$

Etape b) :

$$R'C(O)X \text{ ou } (R'CO)_2O + ROOM \rightarrow R'\text{-}C(O)\text{-}O\text{-}O\text{-}R$$

dans lesquelles :

ROOH est un hydroxy-hydroperoxyde ;
ROOM est un sel d'hydroxy-hydroperoxyde ;
R'C(O)X est un halogénure d'acide ;
$(R'CO)_2O$ est un anhydride d'acide.

Un avantage du procédé selon l'invention est que l'étape b) peut être mise en oeuvre dans le réacteur ayant servi à la réalisation de l'étape a).

Etape a)

L'hydroperoxyde de départ ROOH est un hydroxy-hydroperoxyde.
Comme hydroxy-hydroperoxydes, on utilise en particulier ceux dont le groupement hydroxy est situé en position 3 par rapport au groupement hydroperoxy.
En outre, il est préférable d'utiliser des hydroperoxydes d'hydroxy-alkyle tertiaire.
En tant qu'exemples de tels composés, on peut citer ceux répondant à la formule générale suivante :

$$OH\text{-}C(R_3)(R_4)\text{-}CH_2\text{-}C(R_1)(R_2)\text{-}OOH$$

dans laquelle :

$R_1$ et $R_2$ sont indépendamment l'un de l'autre, un alkyle ayant de 1 à 4 atomes de carbone ;
$R_3$ et $R_4$ sont indépendamment l'un de l'autre, un hydrogène ou un alkyle ayant de 1 à 4 atomes de carbone ;
$R_1$ et $R_3$ peuvent être reliés l'un à l'autre par un pont alkylène ayant 3 atomes de carbone, ce pont étant éventuellement substitué par un alkyle ayant de 1 à 4 atomes de carbone ; et $R_3$ peut en outre être un groupement
$-CH_2\text{-}C(R_1)(R_2)\text{-}OOH$, $R_1$ et $R_2$ étant tels que définis ci-dessus.

Comme hydroperoxydes de hydroxy-t-alkyle, on peut citer les hydroperoxydes de 3-hydroxy-1,1-diméthylpropyle, de 3-hydroxy-1,1-diméthylbutyle, de 1-éthyl-3-hydroxy-1-méthylpentyle, de 1,1-diéthyl-3-hydroxybutyle et de 5-hydroxy-1,3,3-triméthylcyclohexyle.
Selon l'invention, les hydroxy-hydroperoxydes préférés sont les hydroperoxydes d'hexylène glycol, en particulier l'hydroperoxyde de 3-hydroxy-1,1-diméthyl butyle.
Ces hydroperoxydes de hydroxy-t-alkyle peuvent être préparés en traitant les hydroxy-t-alcools correspondants avec un excès de peroxyde d'hydrogène en présence d'un catalyseur fortement acide, tel que l'acide sulfurique, l'acide phosphorique, l'acide perchlorique, l'acide p-toluène sulfonique.
Par exemple, l'hydroperoxyde d'hexylène glycol peut être préparé de cette manière à partir de l'hexylène glycol du commerce, selon les enseignements du brevet américain n° 3 236 872.
Les hydroxy-t-alcools peuvent à leur tour être préparés de façon connue.
Comme base pour l'étape a) du procédé, on peut utiliser des bases minérales telles que NaOH, KOH, LiOH, $Na_2CO_3$, $K_2CO_3$, $NaHCO_3$, $KHCO_3$, Ca$(OH)_2$, Ba$(OH)_2$, $CaCO_3$, $Na_3PO_4$, ou bien des bases organiques telles que des amines comme la pyridine, la N,N-diméthylaniline, la 4-(N,N-diméthylamino)pyridine), la triéthylamine, la tributylamine, le 1-azabicyclo [2.2.2] octane, le 1-4-diazabicylco [2.2.2] octane, le 1,8-diazabicyclo[5.3.0] undec-7-ène, l'urée et la tétraméthyl-urée.
On utilise de préférence une base, en particulier l'hydroxyde de potassium ou de sodium. L'hydroxyde de potassium est la base préférée.
Le rapport molaire $R_a$ entre la base et l'hydroperoxyde est généralement compris entre 0,5 et 1,5.
Selon une mode de réalisation avantageux de l'invention, le rapport molaire $R_a$ est compris entre 0,9 et 1,3 préférentiellement entre 1,00 et 1,22, et en particulier entre 1,10 et 1,19.
L'étape a) est généralement conduite à une température de 20-25°C, l'hydroxy hydroperoxyde étant en général mis à réagir tel quel (à l'état sensiblement pur et sans dissolution préalable dans un solvant organique), progressivement, sous agitation, avec la base minérale, celle-ci se trouvant éventuellement sous forme de solution aqueuse.
Puis, le milieu réactionnel est généralement maintenu sous agitation pendant quelques minutes, pour achever la formation du sel d'hydroxy hydroperoxyde.

Etape b)

Cette étape est mise en oeuvre entre le sel d'hydroperoxyde obtenu au terme de l'étape a) et, soit un anhydride d'acide, soit un halogénure d'acide.
L'anhydride d'acide peut être choisi dans le groupe constitué par les anhydrides des acides 2-méthoxy-propionique, isobutyrique, tertiobutyrique, pivalique, de 2,2-diméthyl butyrique, de 2-éthyl butyrique, hexanoïque, néohexanoïque, benzoïque, heptanoïque, néoheptanoique, 2-éthyl-hexanoïque, octanoïque, néooctanoïque, 2-phénoxy-propanoïque, 2-phényl-

propanoïque, nonanoïque, d'isononanoïque, néononanoïque, 2-méthyl-2-phényl-propionique, 2-phényl butyrique, décanoïque, néodécanoïque, dodécanoïque, 2-butyl octanoïque, néododécanoïque, undécanoïque, néotridécanoïque, méthacrylique, méthyl-crotonique et 2-méthyl-2-buténoïque.

Selon un mode de réalisation préféré de l'invention, l'étape b) est mise en oeuvre avec un halogénure d'acide.

Cet halogénure d'acide a généralement pour formule brute la formule R'COX, dans laquelle :

- R' répond à l'une des formules suivantes :

$$R_5R_6R_7C- \quad \text{et} \quad R_9CH=CR_8-$$

  dans lesquelles :

  $R_5$ est un hydrogène ou un alkyle ayant de 1 à 8 atomes de carbone ;
  $R_6$ est un alkyle ayant de 1 à 8 atomes de carbone ;
  $R_7$ est un alkyle ayant de 1 à 8 atomes de carbone, un alcényle ayant de 1 à 8 atomes de carbone, un aryle ayant de 6 à 10 atomes de carbone, un alcoxy ayant de 1 à 6 atomes de carbone ou un aryloxy ayant de 6 à 10 atomes de carbone ;
  $R_8$ et $R_9$ sont indépendamment l'un de l'autre, un alkyle ayant de 1 à 4 atomes de carbone ; et

- X est un halogène.

Comme halogénures d'acide, on peut donc mettre en oeuvre les halogénures de 2-méthoxy-propionyle, d'isobutyroyle, de tertiobutyroyle, de pivaloyle, de 2,2-diméthyl butyroyle, de 2-éthyl butyroyle, d'hexanoyle, de néohexanoyle, de benzoyle, d'heptanoyle, de néoheptanoyle, de 2-éthyl-hexanoyle, d'octanoyle, de néooctanoyle, de 2-phénoxy-propanoyle, de 2-phényl-propanoyle, de nonanoyle, d'isononanoyle, de néononanoyle, de 2-méthyl-2-phényl-propionyle, de 2-phényl butyroyle, de décanoyle, de néodécanoyle, de dodécanoyle, de 2-butyl octanoyle, de néododécanoyle, de undécanoyle, de néotridécanoyle, de méthacryloyle, de méthyl-crotonoyle et de 2-méthyl-2-buténoyle.

De préférence, l'halogène X est un atome de chlore.

Selon l'invention, l'halogénure d'acide le plus intéressant est le chlorure de néodécanoyle de formule brute t-$C_9H_{19}$-COCl.

Les chlorures d'acides peuvent être préparés de façon connue, par exemple à partir des acides correspondants, par réaction avec des agents de chloration tels que PCl$_3$, POCl$_3$, PCl$_5$, SOCl$_2$, le phosgène (en présence de N,N-diméthylformamide) ou le trichlorobenzène, puis par séparation du chlorure d'acide du milieu réactionnel.

Selon un mode de réalisation avantageux de l'invention, le rapport molaire $R_b$ entre l'hydroxy-hydroperoxyde (correspondant au sel d'hydroperoxyde) et l'halogénure d'acide est compris entre 0,9 et 1,2, préférentiellement entre 1,00 et 1,17 et en particulier entre 1,10 et 1,16.

Dans l'étape b) l'anhydride ou l'halogénure d'acide peut être ajouté tel quel (c'est-à-dire sans dissolution préalable dans un solvant organique) au sel obtenu au terme de l'étape a), par exemple, en 5 à 60 minutes, de préférence en 10 à 20 minutes, et en général sous agitation. La température de début d'addition est de 15 à 25°C et de préférence de 18 à 23°C.

L'addition est généralement assez exothermique et on passe en général d'une température de 20°C à 30°C. Ensuite, la température de réaction est en général maintenue entre 20 et 40°C et de préférence entre 25 et 35°C. De tels intervalles de températures de réaction suffisent en général pour obtenir une bonne cinétique de réaction sans éviter la dégradation du peroxyester formé. Le temps de réaction est généralement de 10 à 90 minutes, et de préférence de 20 à 40 minutes.

Le traitement du milieu réactionnel se fait ensuite en général à température ambiante.

Divers étapes supplémentaires peuvent être mises en oeuvre.

Un premier lavage à l'eau est réalisé, la phase aqueuse est séparée de la phase organique puis éliminée du milieu. Un autre lavage avec une solution aqueuse d'hydroxyde de potassium (ou de sodium) à 3% est opéré, la phase aqueuse est séparée de la phase organique puis éliminée du milieu.

On obtient ainsi le peroxyester qui constitue majoritairement la phase organique.

Celle-ci peut ensuite être dosée par une méthode iodométrique pour déterminer la teneur en peroxyester. La teneur en eau peut être également mesurée de façon approximative par addition d'heptane dans la phase organique puis de façon plus précise par une méthode Karl-Fisher.

## Utilisations

Les peroxyesters et en particulier le peroxynéodécanoate de 1,1-diméthyl-3-hydroxybutyle trouvent de nombreuses applications dans l'industrie.

Ils peuvent intervenir, notamment, dans des réactions de polymérisation, par exemple, dans la polymérisation du chlorure de vinyle, ou dans le thermodurcissage des résines polyester.

On peut se référer aux documents suivants pour trouver des exemples d'applications des peroxyesters :

- la demande internationale n° WO 99/31194 qui traite de la polymérisation des monomères vyniliques ;
- le brevet américain n° 5 612 426 qui concerne la production de PVC ;

- la demande de brevet japonais n° JP 7258316 qui se rapporte à la polymérisation en suspension du chlorure de vinyle ;
- la demande de brevet japonais n° JP 7258315 qui a trait à la préparation d'une émulsion aqueuse ; et
- les demandes de brevet japonais n° JP 7252308 et JP 7252307 qui décrivent la préparation de PVC.

## Exemples

Les exemples suivants illustrent la présente invention sans toutefois en limiter la portée.

Ils décrivent la synthèse du peroxynéodécanoate de 1,1-diméthyl-3-hydroxybutyle.

## Exemple 1

On charge un réacteur double-enveloppe de 250 ml, muni d'une agitation mécanique, d'un thermomètre et connecté à un cryothermostat, avec 62,42 g d'une solution aqueuse à 23% de KOH.

Tout en maintenant la température du réacteur à 20°C, on ajoute progressivement 37,96 g d'hydroperoxyde d'hexylène glycol ayant une pureté de 78%. On agite ensuite le mélange pendant deux minutes à 20°C à une vitesse d'agitation de 200 tours par minute.

Puis, on ajoute dans le réacteur 36,94 g de chlorure de néodécanoyle ayant une pureté de 99,9% sous une agitation de 250 tours minute. La température passe de 20°C au début de l'addition à 26,9°C à la fin de l'addition.

On règle ensuite le cryothermostat à 30°C et la masse réactionnelle est agitée pendant 30 minutes à 30°C à une vitesse d'agitation de 250 tours par minute.

Puis, on règle le cryothermostat à 20°C, on ajoute 24,7 g d'eau et on laisse reposer le milieu réactionnel pendant 5 minutes, ce qui permet d'éliminer du milieu la phase aqueuse. Le milieu est ensuite lavé avec 64,6 g d'une solution aqueuse à 3% de KOH à une vitesse d'agitation de 280 tours par minute pendant 5 minutes.

Après séparation des phases et élimination de la phase aqueuse, on obtient 49,1 g d'une phase organique.

Une analyse iodométrique permet de mesurer une pureté de 91,4% en peroxyester ; le produit contient environ 2,1% d'eau, ce qui conduit à une pureté du produit de 93,3% (en ne considérant pas l'eau comme une impureté). Le rendement, basé sur le chlorure de néodécanoyle, est de 82,3%.

## Exemple 2

On charge un réacteur double-enveloppe de 250 ml, muni d'une agitation mécanique, d'un thermomètre et connecté à un cryothermostat, avec 62,40 g d'une solution aqueuse à 23% de KOH.

Tout en maintenant la température du réacteur à 20°C, on ajoute progressivement 38,28 g d'hydroperoxyde d'hexylène glycol ayant une pureté de 75%. On agite ensuite le mélange pendant deux minutes à 20°C à une vitesse d'agitation de 200 tours par minute.

Puis, on ajoute dans le réacteur 36,93 g de chlorure de néodécanoyle ayant une pureté de 95,5% sous une agitation de 250 tours minute. La température passe de 20°C, au début de l'addition, à 30,3°C à la fin de l'addition.

On règle ensuite le cryothermostat à 30°C et la masse réactionnelle est agitée pendant 30 minutes à 30°C à une vitesse d'agitation de 250 tours par minute.

Puis, on réalise un traitement avec 2 g de peroxyde d'hydrogène à 50% et on agite le milieu réactionnel à 30°C pendant 15 minutes.

On règle ensuite le cryothermostat à 20°C, on ajoute 24,4 g d'eau et on laisse reposer le milieu réactionnel pendant 5 minutes, ce qui permet d'éliminer du milieu la phase aqueuse. Le milieu est ensuite lavé avec 64,9 g d'une solution aqueuse à 3% de KOH à une vitesse d'agitation de 280 tours par minute pendant 5 minutes.

Après séparation des phases et élimination de la phase aqueuse, on obtient 44,9 g d'une phase organique.

Une analyse iodométrique permet de mesurer une pureté de 78,2% en peroxyester ; le produit contient environ 8,9% d'eau, ce qui conduit à une pureté du produit de 85,9% (en ne considérant pas l'eau comme une impureté). Le rendement, basé sur le chlorure de néodécanoyle, est de 72,2%.

## Exemple 3 (comparatif)

On charge un réacteur double-enveloppe de 250 ml, muni d'une agitation mécanique, d'un thermomètre et connecté à un cryothermostat, avec 65 g d'une solution aqueuse à 30% de KOH.

Tout en maintenant la température du réacteur à 20°C, on ajoute progressivement 36,88 g d'hydroperoxyde d'hexylène glycol ayant une pureté de 78%. On agite ensuite le mélange pendant deux minutes à 20°C à une vitesse d'agitation de 200 tours par minute.

Puis, on ajoute dans le réacteur 36,94 g de chlorure de néodécanoyle ayant une pureté de 99,9% sous une agitation de 250 tours minute. La température passe de 20°C, au début de l'addition, à 28°C à la fin de l'addition.

On règle ensuite le cryothermostat à 30°C et la masse réactionnelle est agitée pendant 30 minutes à 30°C à une vitesse d'agitation de 250 tours par minute.

On règle ensuite le cryothermostat à 20°C, on ajoute 24,5 g d'eau et on laisse reposer le milieu réactionnel pendant 5 minutes, ce qui permet d'éliminer du milieu la phase aqueuse. Le milieu est ensuite lavé avec 64,6 g d'une solution aqueuse à 3% de KOH à une vitesse d'agitation de 280 tours par minute pendant 5 minutes.

Aucune séparation des phases n'est observée.

## Revendications

1. Procédé de préparation d'un hydroxy-peroxyester comprenant la préparation d'un sel d'hydroxy-hydroperoxyde par réaction de l'hydroxy-hydroperoxyde correspondant avec une base puis la réaction du sel d'hydroxy-hydroperoxyde avec un halogénure d'acide ou un anhydride d'acide, dans lequel:

   - le sel d'hydroxy-hydroperoxyde est préparé selon un rapport molaire $R_a$ entre la base et l'hydroxy-hydroperoxyde compris entre 0,5 et 1,5; et

   - l'hydroxy-peroxyester est préparé avec un rapport molaire $R_b$ entre l'hydroxy-hydroperoxyde et l'halogénure d'acide compris entre 0,5 et 1,5 ; ou bien un rapport molaire $R_b'$ entre l'hydroxy-hydroperoxyde et l'anhydride d'acide compris entre 1,0 et 3,0 ;

   - ladite réaction du sel d'hydroxy-hydroperoxyde avec un halogénure d'acide ou un anhydride d'acide étant mise en oeuvre en milieux aqueux en l'absence de solvant organique.

2. Procédé selon la revendication 1, **caractérisé en ce que** l'hydroxy-hydroperoxyde est un hydroperoxyde d'hydroxy-alkyle tertiaire.

3. Procédé selon la revendication 1 ou la revendication 2, **caractérisé en ce que** l'hydroxy-hydroperoxyde est un hydroperoxyde d'hydroxy-alkyle tertiaire dont le groupement hydroxy est situé en position 3 par rapport au groupement hydroperoxy.

4. Procédé selon la revendication 3, **caractérisé en ce que** l'hydroxy-hydroperoxyde répond à la formule suivante :

   $$OH\text{-}C(R_3)(R_4)\text{-}CH_2\text{-}C(R_1)(R_2)\text{-}OOH$$

   dans laquelle :

   $R_1$ et $R_2$ sont indépendamment l'un de l'autre, un alkyle ayant de 1 à 4 atomes de carbone ;
   $R_3$ et $R_4$ sont indépendamment l'un de l'autre, un hydrogène ou un alkyle ayant de 1 à 4 atomes de carbone ;
   $R_1$ et $R_3$ peuvent être reliés l'un à l'autre par un pont alkylène ayant 3 atomes de carbone, ce pont étant éventuellement substitué par un alkyle ayant de 1 à 4 atomes de carbone et $R_3$ peut

   en outre être un groupement
   $-CH_2\text{-}C(R_1)(R_2)\text{-}OOH$.

5. Procédé selon la revendication 4, **caractérisé en ce que** l'hydroperoxyde d'hydroxy-alkyle tertiaire est choisi dans le groupe constitué par les hydroperoxydes de 3-hydroxy-1,1-diméthylpropyle, de 3-hydroxy-1,1-diméthylbutyle, de 1-éthyl-3-hydroxy-1-méthylpentyle, de 1,1-diéthyl-3-hydroxybutyle et de 5-hydroxy-1,3,3-triméthylcyclohexyle.

6. Procédé selon l'une des revendications 1 à 5, **caractérisé en ce qu'**il est mis en oeuvre en l'absence de catalyseur de transfert de phase.

7. Procédé selon l'une des revendications 1 à 6, **caractérisé en ce que** la base est KOH.

8. Procédé selon l'une des revendications 1 à 7, **caractérisé en ce que** le rapport $R_a$ est compris entre 0,9 et 1,3 préférentiellement entre 1,00 et 1,22 et en particulier entre 1,10 et 1,19.

9. Procédé selon l'une des revendications 1 à 8, **caractérisé en ce que** l'anhydride d'acide est choisi dans le groupe constitué par les anhydrides des acides 2-méthoxy-propionique, isobutyrique, tertiobutyrique, pivalique, de 2,2-diméthyl butyrique, de 2-éthyl butyrique, hexanoïque, néohexanoïque, benzoïque, heptanoïque, néoheptanoique, 2-éthylhexanoïque, octanoïque, néooctanoïque, 2-phénoxy-propanoïque, 2-phényl-propanoïque, nonanoïque, isononanoïque, néononanoïque, 2-méthyl-2-phényl-propionique, 2-phényl butyrique, décanoïque, néodécanoïque, dodécanoïque, 2-butyl octanoïque, néododécanoïque, undécanoïque, néotridécanoïque, méthacrylique, méthyl-crotonique et 2-méthyl-2-buténoïque.

10. Procédé selon l'une des revendications 1 à 9, **caractérisé en ce que** la réaction est effectuée entre un sel d'hydroxy-hydroperoxyde et un halogénure d'acide.

11. Procédé selon la revendication 10, **caractérisé en ce que** l'halogénure d'acide répond à la formule R'COX, dans laquelle :

   - R' répond à l'une des formules suivantes :

     $$R_5R_6R_7C\text{-} \quad et \quad R_9CH{=}CR_8\text{-}$$

     dans lesquelles :

     $R_5$ est un hydrogène ou un alkyle ayant de 1 à 8 atomes de carbone ;
     $R_6$ est un alkyle ayant de 1 à 8 atomes de

carbone ;

R$_7$ est un alkyle ayant de 1 à 8 atomes de carbone, un alcényle ayant de 2 à 8 atomes de carbone, un aryle ayant de 6 à 10 atomes de carbone, un alcoxy ayant de 1 à 6 atomes de carbone ou un aryloxy ayant de 6 à 10 atomes de carbone ;

R$_8$ et R$_9$ sont, indépendamment l'un de l'autre, un alkyle ayant de 1 à 4 atomes de carbone ; et

- X est un halogène.

12. Procédé selon la revendication 11, **caractérisé en ce que** l'halogénure est choisi dans le groupe constitué par les halogénures de 2-méthoxy-propionyle, d'isobutyroyle, de tertiobutyroyle, de pivaloyle, de 2,2-diméthyl butyroyle, de 2-éthyl butyroyle, d'hexanoyle, de néohexanoyle, de benzoyle, d'heptanoyle, de néoheptanoyle, de 2-éthyl-hexanoyle, d'octanoyle, de néooctanoyle, de 2-phénoxy-propanoyle, de 2-phényl-propanoyle, de nonanoyle, d'isononanoyle, de néononanoyle, de 2-méthyl-2-phényl-propionyle, de 2-phényl butyroyle, de décanoyle, de néodécanoyle, de dodécanoyle, de 2-butyl octanoyle, de néododécanoyle, de undécanoyle, de néotridécanoyle, de méthacryloyle, de méthylcrotonoyle et de 2-méthyl-2-buténoyle.

13. Procédé selon la revendication 12, **caractérisé en ce que** l'halogénure d'acide est un chlorure d'acide.

14. Procédé selon la revendication 13, **caractérisé en ce que** l'halogénure d'acide est le chlorure de néodécanoyle.

15. Procédé selon l'une des revendications 1 à 14, **caractérisé en ce que** le rapport R$_b$ est compris entre 0,9 et 1,2, préférentiellement entre 1,00 et 1,17 et en particulier entre 1,10 et 1,16.

16. Procédé selon l'une des revendications 1 à 15, **caractérisé en ce que** l'hydroxy-hydroperoxyde est l'hydroperoxyde de 3-hydroxy-1,1-diméthyl butyle et l'halogénure d'acide est le chlorure de néodécanoyle.

**Patentansprüche**

1. Verfahren zur Herstellung eines Hydroxyperoxyesters aufweisend die Herstellung eines Hydroxyhydroperoxid-Salzes durch Reaktion des entsprechenden Hydroxyhydroperoxids mit einer Base, dann die Reaktion des Hydroxyhydroperoxid-Salzes mit einem Säurehalogenid oder einem Säureanhydrid, bei dem:

- das Hydroxyhydroperoxid-Salz mit einem Molverhältnis R$_a$ zwischen der Base und dem Hydroxyhydroperoxid zwischen 0,5 und 1,5 hergestellt wird; und

- der Hydroxyperoxyester mit einem Molverhältnis R$_b$ zwischen dem Hydroxyhydroperoxid und dem Säurehalogenid zwischen 0,5 und 1,5; oder aber einem Molverhältnis R$_b$' zwischen dem Hydroxyhydroperoxid und dem Säureanhydrid zwischen 1,0 und 3,0 hergestellt wird;

- wobei die Reaktion des Hydroxyhydroperoxid-Salzes mit einem Säurehalogenid oder einem Säureanhydrid im wässerigen Milieu in Abwesenheit von organischem Lösungsmittel durchgeführt wird.

2. Verfahren nach Anspruch 1, **dadurch gekennzeichnet, dass** das Hydroxyhydroperoxid ein tertiäres Hydroxyalkyl-hydroperoxid ist.

3. Verfahren nach Anspruch 1 oder Anspruch 2, **dadurch gekennzeichnet, dass** das Hydroxyhydroperoxid ein tertiäres Hydroxyalkyl-hydroperoxid ist, dessen Hydroxy-Gruppe sich bezüglich der Hydroperoxy-Gruppe in Position 3 befindet.

4. Verfahren nach Anspruch 3, **dadurch gekennzeichnet, dass** das Hydroxyhydroperoxid der folgenden Formel entspricht:

$$OH-C(R_3)(R_4)-CH_2-C(R_1)(R_2)-OOH$$

in der:

R$_1$ und R$_2$ jeweils unabhängig voneinander ein Alkyl mit 1 - 4 Kohlenstoffatomen sind;

R$_3$ und R$_4$ jeweils unabhängig voneinander Wasserstoff oder ein Alkyl mit 1 bis 4 Kohlenstoffatomen sind;

R$_1$ und R$_3$ durch eine Alkylen-Brücke mit drei Kohlenstoffatomen miteinander verbunden sein können, wobei diese Brücke gewünschtenfalls mit einem Alkyl mit 1 bis 4 Kohlenstoffatomen substituiert sein kann und R$_3$ außerdem eine Gruppe -CH$_2$-C(R$_1$)(R$_2$)-OOH sein kann.

5. Verfahren nach Anspruch 4, **dadurch gekennzeichnet, dass** das tertiäre Hydroxyalkyl-hydroperoxid ausgewählt wird aus der Gruppe, die besteht aus 3-Hydroxy-1,1-dimethylpropyl-hydroperoxid, 3-Hydroxy-1,1-dimethylbutyl-hydroperoxid, 1-Ethyl-3-hydroxy-1-methylpentyl-hydroperoxid, 1,1-Diethyl-3-hydroxybutyl-hydroperoxid und 5-Hydroxy-1,3,3-trimethylcyclohexyl-hydroperoxid.

6. Verfahren nach einem der Ansprüche 1 bis 5, **dadurch gekennzeichnet, dass** es in Abwesenheit eines Phasentransferkatalysators durchgeführt wird.

7. Verfahren nach einem der Ansprüche 1 bis 6, **dadurch gekennzeichnet, dass** die Base KOH ist.

8. Verfahren nach einem der Ansprüche 1 bis 7, **dadurch gekennzeichnet, dass** das Verhältnis $R_a$ zwischen 0,9 und 1,3, bevorzugt zwischen 1,00 und 1,22, und insbesondere zwischen 1,10 und 1,19, liegt.

9. Verfahren nach einem der Ansprüche 1 bis 8, **dadurch gekennzeichnet, dass** das Säureanhydrid ausgewählt wird aus der Gruppe, die besteht aus den Anhydriden der Säuren 2-Methoxypropionsäure, Isobutansäure, Tertiärbutansäure, Pivalinsäure, 2,2-Dimethyl-butansäure, 2-Ethyl-butansäure, Hexansäure, Neohexansäure, Benzoesäure, Heptansäure, Neoheptansäure, 2-Ethylhexansäure, Octansäure, Neooctansäure, 2-Phenoxy-propansäure, 2-Phenyl-propansäure, Nonansäure, Isononansäure, Neononansäure, 2-Methyl-2-phenyl-propionsäure, 2-Phenyl-butansäure, Decansäure, Neodecansäure, Dodecansäure, 2-Butyl-octansäure, Neododecansäure, Undecansäure, Neotridecansäure, Methacrylsäure, Methyl-crotonsäure und 2-Methyl-2-butensäure.

10. Verfahren nach einem der Ansprüche 1 bis 9, **dadurch gekennzeichnet, dass** die Reaktion zwischen einem Hydroxyhydroperoxid-Salz und einem Säurehalogenid bewirkt wird.

11. Verfahren nach Anspruch 10, **dadurch gekennzeichnet, dass** das Säurehalogenid der Formel R'COX entspricht, in der:

- R' einer der folgenden Formeln entspricht:

$$R_5R_6R_7C\text{- und } R_9CH=CR_8\text{-}$$

in denen:

$R_5$ Wasserstoff oder ein Alkyl mit 1 - 8 Kohlenstoffatomen ist;

$R_6$ ein Alkyl mit 1 - 8 Kohlenstoffatomen ist;

$R_7$ ein Alkyl mit 1 - 8 Kohlenstoffatomen, ein Alkenyl mit 2-8 Kohlenstoffatomen, ein Aryl mit 6-10 Kohlenstoffatomen, ein Alkoxy mit 1 - 6 Kohlenstoffatomen oder ein Aryloxy mit 6 - 10 Kohlenstoffatomen ist;

$R_8$ und $R_9$ jeweils unabhängig voneinander ein Alkyl mit 1 - 4 Kohlenstoffatomen sind; und

- X ein Halogen ist.

12. Verfahren nach Anspruch 11, **dadurch gekennzeichnet, dass** das Halogenid ausgewählt wird aus der Gruppe, die besteht aus 2-Methoxy-propionyl-halogenid, Isobutyroyl-halogenid, Tertiärbutyroyl-halogenid, Pivaloyl-halogenid, 2,2-Dimethyl-butyroyl-halogenid, 2-Ethyl-butyroyl-halogenid, Hexanoyl-halogenid, Neohexanoyl-halogenid, Benzoyl-halogenid, Heptanoylhalogenid, Neoheptanoyl-halogenid, 2-Ethyl-hexanoyl-halogenid, Octanoyl-halogenid, Neooctanoyl-halogenid, 2-Phenoxy-propanoyl-halogenid, 2-Phenyl-propanoyl-halogenid, Nonanoyl-halogenid, Isononanoyl-halogenid, Neononanoyl-halogenid, 2-Methyl-2-phenyl-propionyl-halogenid, 2-Phenyl-butyroyl-halogenid, Decanoyl-halogenid, Neodecanoyl-halogenid, Dodecanoyl-halogenid, 2-Butyl-octanoyl-halogenid, Neododecanoylhalogenid, Undecanoyl-halogenid, Neotridecanoyl-halogenid, Methacryloylhalogenid, Methylcrotonoyl-halogenid und 2-Methyl-2-butenoyl-halogenid.

13. Verfahren nach Anspruch 12, **dadurch gekennzeichnet, dass** das Säurehalogenid ein Säurechlorid ist.

14. Verfahren nach Anspruch 13, **dadurch gekennzeichnet, dass** das Säurehalogenid Neodecanoylchlorid ist.

15. Verfahren nach einem der Ansprüche 1 bis 14, **dadurch gekennzeichnet, dass** das Verhältnis $R_b$ zwischen 0,9 und 1,2, bevorzugt zwischen 1,00 und 1,17, und insbesondere zwischen 1,10 und 1,16, liegt.

16. Verfahren nach einem der Ansprüche 1 bis 15, **dadurch gekennzeichnet, dass** das Hydroxyhydroperoxid 3-Hydroxy-1,1-dimethyl-butyl-hydroxyperoxid ist und das Säurehalogenid Neodecanoylchlorid ist.

## Claims

1. Process for the preparation of a hydroxyperoxyester comprising the preparation of a hydroxyhydroperoxide salt by reaction of the corresponding hydroxyhydroperoxide with a base, then reacting the hydroxyhydroperoxide salt with an acid halide or an acid anhydride, in which :

- the hydroxyhydroperoxide salt is prepared according to a molar ratio $R_a$ of the base to the hydroxy-hydroperoxide of between 0.5 and 1.5; and
- the hydroxyperoxyester is prepared according to a molar ratio $R_b$ of the hydroxyhydroperoxide corresponding to the hydroxyhydroperoxide salt to the acid halide is between 0.5 and 1.5; or alternatively the molar ratio $R_b'$ of the hydroxyhydroperoxide corresponding to the hydroxyhydroperoxide salt to the acid anhydride is between 1.0 and 3.0;
- the said reacting of the hydroxyhydroperoxide salt with an acid halide or an acid anhydride is carried out in aqueous media, in the absence of organic solvent.

2. Process according to Claim 1, **characterized in that** the hydroxyhydroperoxide is a hydroxy(tertiairyalkyl) hydroperoxide.

3. Process according to Claim 1 or Claim 2, **characterized in that** the hydroxyhydroperoxide is a hydroxy(tertiary-alkyl) hydroperoxide in which the hydroxyl group is situated in the 3 position with respect to the hydroperoxy group.

4. Process according to Claim 3, **characterized in that** the hydroxyhydroperoxide corresponds to the following formula :

$$HO-C(R_3)(R_4)-CH_2-C(R_1)(R_2)-OOH$$

in which :

$R_1$ and $R_2$ are, independently of one another, an alkyl having from 1 to 4 carbon atoms; $R_3$ and $R_4$ are, independently of one another, a hydrogen or an alkyl having from 1 to 4 carbon atoms; $R_1$ and $R_3$ can be connected to one another via an alkylene bridge having 3 carbon atoms, this bridge optionally being substituted by an alkyl having from 1 to 4 carbon atoms; and $R_3$ can additionally be a $-CH_2-C(R_1)(R_2)-OOH$ group.

5. Process according to Claim 4, **characterized in that** the hydroxy(tertiary-alkyl) hydroperoxide is chosen from the group consisting of 3-hydroxy-1,1-dimethylpropyl, 3-hydroxy-1,1-dimethylbutyl, 1-ethyl-3-hydroxy-1-methylpentyl, 1,1-diethyl-3-hydroxybutyl and 5-hydroxy-1,3,3-trimethylcyclohexyl hydroperoxides.

6. Process according to one of Claims 1 to 5, **charac-**

terized in that it is carried out in the absence of phase transfer catalyst.

7. Process according to one of Claims 1 to 6, **characterized in that** the base is KOH.

8. Process according to one of Claims 1 to 7, **characterized in that** the ratio $R_a$ is between 0.9 and 1.3, preferably between 1.00 and 1.22 and in particular between 1.10 and 1.19.

9. Process according to one of Claims 1 to 8, **characterized in that** the acid anhydride is chosen from the group consisting of anhydrides of 2-methoxypropionic, isobutyric, tert-butyric, pivalic, 2,2-dimethylbutyric, 2-ethylbutyric, hexanoic, neohexanoic, benzoic, heptanoic, neoheptanoic, 2-ethylhexanoic, octanoic, neooctanoic, 2-phenoxypropanoic, 2-phenylpropanoic, nonanoic, isononanoic, neononanoic, 2-methyl-2-phenylpropionic, 2-phenylbutyric, decanoic, neodecanoic, dodecanoic, 2-butyloctanoic, neododecanoic, undecanoic, neotridecanoic, methacrylic, methylcrotonic and 2-methyl-2-butenoic acids.

10. Process according to one of Claims 1 to 9, **characterized in that** the reaction is carried out between a hydroxyhydroperoxide salt and an acid halide.

11. Process according to Claim 10, **characterized in that** the acid halide corresponds to the formula R'COX, in which :

- R' corresponds to one of the following formulae :

$$R_5R_6R_7C- \qquad and \qquad R_9CH=CR_8-$$

in which :

$R_5$ is a hydrogen or an alkyl having from 1 to 8 carbon atoms; $R_6$ is an alkyl having from 1 to 8 carbon atoms; $R_7$ is an alkyl having from 1 to 8 carbon atoms, an alkenyl having from 2 to 8 carbon atoms, an aryl having from 6 to 10 carbon atoms, an alkoxy having from 1 to 6 carbon atoms or an aryloxy having from 6 to 10 carbon atoms; $R_8$ and $R_9$ are, independently of one another, an alkyl having from 1 to 4 carbon atoms; and

- X is a halogen.

12. Process according to Claim 11, **characterized in**

**that** the halide is chosen from the group consisting of 2-methoxypropionyl, isobutyroyl, tert-butyroyl, pivaloyl, 2,2-dimethylbutyroyl, 2-ethylbutyroyl, hexanoyl, neohexanoyl, benzoyl, heptanoyl, neoheptanoyl, 2-ethylhexanoyl, octanoyl, neooctanoyl, 2-phenoxypropanoyl, 2-phenylpropanoyl, nonanoyl, isononanoyl, neononanoyl, 2-methyl-2-phenylpropionyl, 2-phenylbutyroyl, decanoyl, neodecanoyl, dodecanoyl, 2-butyloctanoyl, neododecanoyl, undecanoyl, neotridecanoyl, methacryloyl, methylcrotonoyl and 2-methyl-2-butenoyl halides.

13. Process according to Claim 12, **characterized in that** the acid halide is an acid chloride.

14. Process according to Claim 13, **characterized in that** the acid halide is neodecanoyl chloride.

15. Process according to one of Claims 1 to 14, **characterized in that** the ratio $R_b$ is between 0.9 and 1.2, preferably between 1.00 and 1.17 and in particular between 1.10 and 1.16.

16. Process according to one of Claims 1 to 15, **characterized in that** the hydroxyhydroperoxide is 3-hydroxy-1,1-dimethylbutyl hydroperoxide and the acid halide is neodecanoyl chloride.